# EUROPEAN PATENT SPECIFICATION

(11) **EP 0 523 406 B1**
(45) Date of publication and mention of the grant of the patent: **09.12.1998**
(21) Application number: 92110522.7
(22) Date of filing: 23.06.1992
(51) Int. Cl.: C12N 7/06

(54) **Use of tri(n-butyl) phosphate at low pH in solutions of biologically active proteins for enhanced virucidal activity**
Verwendung von Tri-n-Butylphosphat bei niedrigem pH in Lösungen von biologisch aktiven Proteinen für eine verbesserte viruzide Wirkung
Utilisation de phosphate de tri-n-butyle à bas pH dans des solutions de protéines biologiquement actives pour l'amélioration de l'activité virucide

(30) Priority: 05.07.1991 US 726086
(43) Date of publication of application: 20.01.1993
(73) Proprietor: Bayer Corporation, Pittsburgh, PA 15205-9741 (US)
(72) Inventor: Dobkin, Milton B., Lafayette, California 94549 (US); Dove, George B., Hercules, California 94547 (US)
(74) Representative: Dänner, Klaus, Dr.

(56) References cited:
- EP-A- 131 740
- EP-A- 0 306 778
- EP-A- 0 378 208
- US-A- 3 962 421
- US-A- 5 071 650
- US-A- 5 110 910
- DATABASE WPIL Week 9034, Derwent Publications Ltd., London, GB; AN 90-257580 & JP-A-2 180 833 (GREEN CROSS CORP) 13 July 1990

## Description

### BACKGROUND OF THE INVENTION

Field: This disclosure is concerned generally with methods of inactivating viruses in solutions of therapeutic biologically active proteins and, specifically, with the use of tri(n-butyl) phosphate (TNBP), under unusual conditions, sufficient to assure virucidal action without adverse effect on the proteins.

Prior Art: The utility of nonexplosive organic solvent mixtures for the preparation of viral vaccines, and, more recently, for inactivation of endogenous viruses in preparations of biological products derived from human plasma, have been limited by the pH sensitivity of the proteins of interest.

One common virucidal compound used with biologically active proteins is TNBP, often combined with a non-ionic detergent such as polysorbate 80 (Tween 80), or an anionic detergent, cholate. Treatment is carried out at neutral pH. See, for example, Horowitz et al, Transfusion 1985; 25:516-522.

EP-A-0 306 778 describes a method for sterilisation of plasma or plasma fractions by treating these materials with Tri-(n-butyl)-phosphate (TNBP) and potassium cholate together with beta-propiolactone and/or UV irradiation at a pH between 4.5 and 8.3.

In the past, biologically active proteins (e.g., obtained from blood) were processed at a neutral pH (i.e., 6.8 to 7.2) and it has been in this pH range that virucidal compounds have been used. The development of biological products (mainly antibodies of IgG and IgM types), from both human plasma origin and hybridoma technology, as well as recombinant DNA products, has recently permitted purification processes at much lower pH without deleterious effects.

We have found that there is a novel pH-dependency of TNBP for inactivating lipid-enveloped viruses while preserving the biological activity of proteins such as immunoglobulins. This pH dependency has been found to be fortuitously consistent with the finding that certain biologically active proteins (e.g., antibodies) can be advantageously processed at lower pH ranges. Details of our discovery are described below.

### SUMMARY OF THE INVENTION

Our method of assuring the substantial reduction (viral inactivation) of lipid-coated viruses in an aqueous solution of biologically active, therapeutic proteins comprises contacting the solution with TNBP but without UV-irradiation or β-propiolactone, at a pH ranging from 3.0 to 5.0, preferably at a pH from 4.0 to 4.85 under conditions sufficient to assure the substantial reduction of viruses (at least 2 logs virus titer reduction) without adversely affecting the biological activity of the proteins (i.e., less than 30 % loss of activity).

### BRIEF DESCRIPTION OF THE FIGURES

Figure 1 is a graph showing the relationship of pH to virus inactivation using the method of this invention.

Figure 2 is a graph showing the effect of pH, as a function of time, on virus inactivation using the method of this invention.

### SPECIFIC EMBODIMENTS

Evidence to support the enhanced virucidal efficacy of TNBP at low pH followed the demonstration of similar pH-dependency of saturated intermediate chain-length alcohols and acids for inactivating lipid-enveloped viruses (see, for example, Dobkin et al, Biologicals 1991; (19(in press)). There is no published literature using TNBP for inactivating viruses at pH below 6.0 (see, for example, Edwards et al, Vox Sanguinis 1987; 52:53-59).

### MATERIALS AND METHODS

### Chemical Agents

Tri(n-butyl) phosphate and the detergent polysorbate 80 (Tween 80) were reagent grade and obtained from Sigma Chemicals, St. Louis, Mo. Protein solutions were buffered at the indicated pH with sodium acetate (J.T. Baker Chemical Co., Phillipsburg, NJ.) and TRIS (hydroxymethyl)aminomethane (Trizma base, Sigma).

### Virus

Vesicular stomatitis virus (VSV), Indiana strain, obtained from the Finnish Red Cross, is a lipid-enveloped Rhabdovirus. Vaccinia virus, Lederle strain, ATCC VR-118, is a lipid-enveloped Poxvirus. Sindbis (Togavirus) and Visna (Lentivirus) are lipid-enveloped viruses. Derivation of viruses was reported previously (see, Lembach et al, Current Studies in Hematology and Blood Transfusion. Basel, Karger, 1989; 56:97-108).

### Virus Assay

VSV was titrated under standard conditions on monolayers of VERO cells grown in 24 well plates using 4 wells per dilution. Titers are expressed in terms of tissue culture infectious doses as a 50% end-point per mL (TCID₅₀/mL).

Other viruses were titrated in similar fashion on monolayers of their respective host cells under standard conditions assessing cytopathic effects.

### Proteins

Plasma-derived IgM (pd-IgM) was purified from human plasma by the Cohn-Oncley process (Fraction III), modified by Steinbuch. Human serum albumin was purified from human plasma by the Cohn-Oncley process (Fraction V).

Monoclonal antibody (human) of class G (m-IgG, anti-tumor necrosis factor, cell line ATCC Accession No. HB9736, was derived from Epstein-Barr virus-transformed human B lymphocytes grown in suspension culture. IgG was purified to greater than 98% by ion exchange and size exclusion chromatography.

Protein recovery was evaluated by A280, radial immunodiffusion, and FPLC-Superose 6 (Pharmacia fast protein liquid chromatography by size exclusion).

### Treatment Protocol

Protein solutions were seeded with a 1/100 dilution of stock virus to minimize the effects of virus-containing medium. A volume of TNBP or TNBP/Tween 80 concentrate was added to give a final concentration of 0.3% TNBP or 0.3% TNBP and 1.0% Tween 80. Treated and control samples were incubated with continuous gentle mixing at 4°C or 24°C under the specified conditions. Samples were removed at appropriate intervals and titrated immediately for residual infectious virus.

### RESULTS

### Effect of pH on Virucidal Efficiency

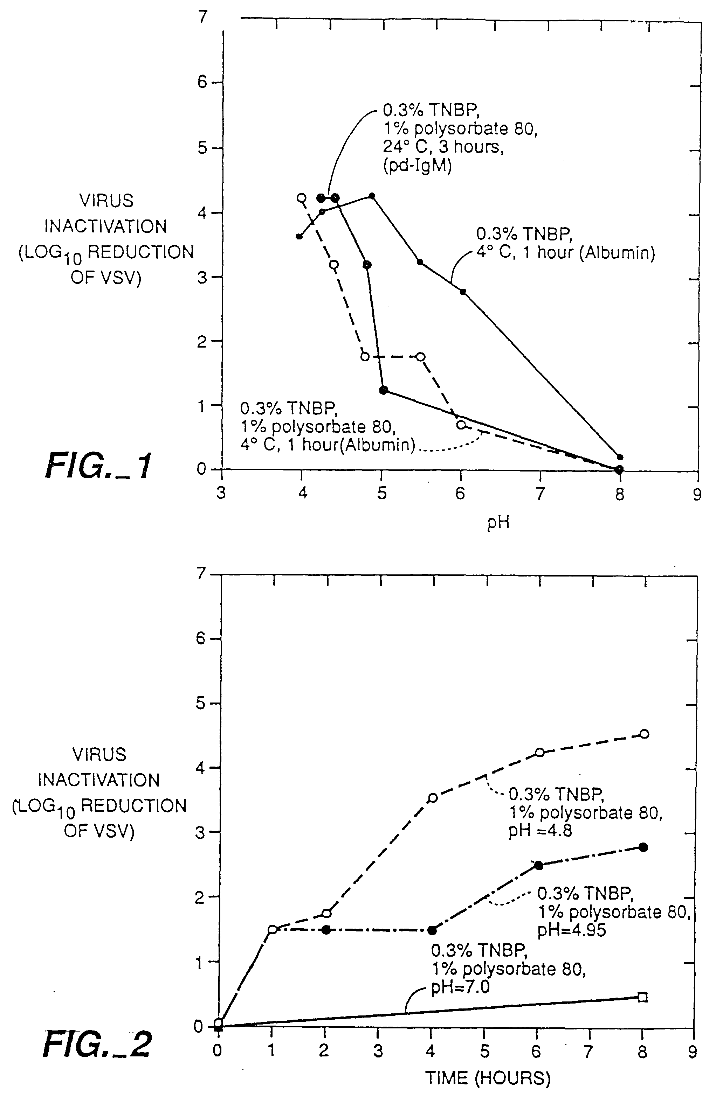

To determine the influence of pH on the ability of TNBP to inactivate viruses in a model protein solution, an experiment was carried out with VSV in a 0.5 mg/mL solution of human albumin at 4°C for 60 minutes. Additionally, in order to assess the virucidal efficiency of the separate components as well as the combination, treatment was carried out with 0.3% TNBP, 1.0% Tween 80, or a mixture of 0.3% TNBP and 1.0% Tween 80. The results of this comparison are shown in **Table 1**. Virus reduction (log₁₀) is plotted as a function of pH, as shown in **Figure 1**.

**Table 1**

| | | | |
|---|---|---|---|
| Effect of pH on Inactivation of VSV in 0.5 mg/ml Human Albumin Containing Various Combinations of 0.3% TNBP and 1.0% Tween 80 at 4°C for 60 minutes. | | | |

| Virus Titer Remaining | | | |
|---|---|---|---|
| pH | TNBP | Tween 80 | TNBP/Tween 80 |
| 4.0 | 3.25 | 6.75 | ≤2.5* |
| 4.4 | 3.0 | N.D. | 3.5 |
| 4.8 | ≤2.75 | ≥7.25 | 5.0 |
| 5.5 | 4.0 | N.D | 5.0 |
| 6.0 | 4.5 | ≥7.5 | 6.0 |
| 8.0 | 6.75 | ≥7.5 | 6.75 |
| | | | |
| Control (No TNBP or Tween 80) | | | |
| | | | |
| 4.0 | 7.0 | 7.25 | 6.75 |

| | | | |
|---|---|---|---|
| *: LOG₁₀TCID₅₀/ML | | | |

In the absence of any inactivating effect of pH, per se, on the virus, the maximum virus kill with TNBP, with or without Tween 80, was obtained at pH 4.0 - 4.8. An inverse relationship was demonstrated between increasing pH and virucidal efficiency. Under the conditions of this experiment, it is clear that TNBP was the essential virucidal component, and Tween 80 provided no enhancement at pH less than 6.0.

In another experiment, the effect of pH on the inactivation kinetics of VSV in a solution of pd-IgM containing 0.3% TNBP and 1.0% Tween 80 was evaluated. Except for the proteins and the virucidal reagents, no salts or other ions were present in this solution. The pH range was 4.25 - 5.1, and the incubation conditions were 24°C for 3 hours.

The effect of pH on viral infectivity is seen in **Table 2**. Virus reduction (log₁₀) in pd-IgM is plotted as a function of pH, as shown in **Figure 1**.

**Table 2**

| | | | | | |
|---|---|---|---|---|---|
| Inactivation Kinetics of VSV in pd-IgM at pH 4.25 - 5.1 Containing 0.3% TNBP and 1.0% Tween 80 or 0.3% TNBP Alone at 24°C for 1 and 3 hours. | | | | | |

| Virus Titer Remaining | | | | | |
|---|---|---|---|---|---|
| Time (hours) | pH | | | | |
| | 4.25 | 4.4 | 4.8 | 5.1 | 4.8 (no Tween) |
| 1 | ≤2.5* | ≤2.5 | 3.5 | 5.5 | ≤2.75 |
| 3 | ≤2.5 | ≤2.5 | ≤2.5 | 5.25 | ≤2.5 |
| | | | | | |

| Control (No TNBP/Tween) | | | | | |
|---|---|---|---|---|---|
| 0 | -- | -- | -- | -- | 7.75 |
| 3 | 6.75 | -- | -- | -- | 7.0 |

| | | | | | |
|---|---|---|---|---|---|
| *: LOG₁₀TCID₅₀/ML --: not tested. | | | | | |

Consistent with the previous data (**Table 1**), VSV was reduced below minimum detection at pH 4.25 and pH 4.4 in less than 1 hour, and at pH 4.8 after 3 hours of treatment. At pH 5.1, virus remained detectable after 3 hours at 24°C, having been reduced in titer only 1.5 logs. Thus, even in an environment of low ionic strength, 0.3% TNBP and 1.0% Tween 80 remained highly virucidal, provided treatment was carried out below pH 5.1. The last column shows that the presence of the polysorbate 80 (Tween 80) is not required; inactivation at lower pH was greater without the Tween 80.

A detailed kinetics study between pH 4.8 and pH 4.95 is given in **Table 3**. Virus reduction is plotted as a function of time in **Figure 2**. Virucidal efficiency was much greater at pH 4.8, compared to pH 4.95 or 7.0.

**Table 3**

| | | | | | |
|---|---|---|---|---|---|
| Inactivation Kinetics of VSV in pd-IgM at 24°C for 8 hours. | | | | | |

| Virus Titer Remaining | | | | | |
|---|---|---|---|---|---|
| Time (Hours) | TNBP | 0.3% | 0.3% | 0.3% | 0.24% |
| | Tween | 0 | 1.0% | 1.0% | 0.8% |
| | pH | 4.8 | 4.8 | 4.95 | 4.85 |
| 1 | | ≤2.75 | 5.5 | 5.75 | 5.75 |
| 2 | | ≤2.5 | 5.25 | 5.75 | -- |
| 4 | | ≤2.5 | 3.5 | 5.75 | 4.75 |
| 6 | | ≤2.5 | ≤2.75 | 4.75 | 4.25 |
| 8 | | ≤2.5 | ≤2.5 | 4.5 | 4.0 |
| | | | | | |

| Control (No TNBP/Tween) | | | | | |
|---|---|---|---|---|---|
| | | | | | |
| 8 | | 7.0 | 7.0 | 7.25 | 7.5 |
| *: LOG₁₀TCID₅₀/ML | | | | | |
| --: not tested. | | | | | |

Studies were conducted to determine whether other viruses were similarly inactivated by TNBP at low pH. The effect of 0.3% TNBP, 1.0% Tween 80 at pH 4.85 is seen in **Table 4**, demonstrating that a variety of viruses are inactivated.

**Table 4**

| | | | | |
|---|---|---|---|---|
| Inactivation Kinetics of VSV in pd-IgM at pH 4.85 Containing 0.3% TNBP and 1.0% Tween 80 at 24°C for 4 hours. | | | | |

| Time | Virus Titer Remaining | | | |
|---|---|---|---|---|
| (hours) | VSV | Sindbis | Vaccinia | Visna |
| 0 | (8.0)* | (6.75) | (4.5) | (6.25) |
| | | | | |
| 0.5 | 3.25 | --- | --- | 4.25 |
| | | | | |
| 1 | 5.25 | ≤2.5 | ≤2.5 | 3.25 |
| | | | | |
| 2 | 4.0 | ≤2.5 | ≤2.5 | ≤2.75 |
| | | | | |
| 4 | ≤2.75 (7.5) | ≤2.5 (6.0) | ≤2.5 (4.0) | ≤2.5 (5.75) |

| | | | | |
|---|---|---|---|---|
| ( ): untreated *: LOG₁₀TCID₅₀/ML --: not tested. | | | | |

Protein recovery under a variety of conditions is reported in **Table 5**. Recovery was not substantially affected under these conditions. It is noted that the effects of low pH alone may be detrimental to specific proteins. However, in the case of the antibodies and other cited proteins, no significant loss of activity was found after using the low pH methods of this disclosure.

**Table 5**

| Effect of Conditions on Protein Recovery in Buffered Solutions Containing 0.3% TNBP and 1.0% Tween 80 at 24°C for 8 hours. | | | |
|---|---|---|---|
| pH | Recovery (% of initial) | | |
| | pd-IgM | Albumin | IgG |
| 4.0 | 85 | 100 | 95 |
| 4.8 | 95 | 100 | 100 |
| 5.5 | 100 | 100 | 100 |

Given the above disclosure it is thought variations will occur to those skilled in the art. Thus, the above examples should be construed as illustrative and the invention disclosed here should be limited only by the following claims.

## Claims

1. A method of inactivating viruses in an aqueous solution of therapeutic, biologically active proteins comprising the steps of contacting the solution with TNBP but without UV-irradiation or β-propiolactone, at a pH ranging from 3.0 to 5.0 under conditions sufficient to assure substantial reduction of the viruses without adverse effect on the proteins.

2. The method of claim 1 wherein the solution is contacted with TNBP and a detergent.

3. The method of claim 2 wherein the detergent is polysorbate 80.

4. The method of claim 1 wherein the pH ranges from about 4.0 to about 4.85.

5. The method of claim 1 wherein the proteins are antibodies.

6. The method of claim 5 wherein the antibodies are of type IgM or IgG.

7. The method of claim 1 wherein reduction of viruses is greater than 2 logs.

8. The method of claim 1 wherein adverse effects on the biological activity of the proteins are less than 30%.

## Patentansprüche

1. Verfahren zur Inaktivierung von Viren in einer wäßrigen Lösung therapeutischer, biologisch wirksamer Proteine, wobei das Verfahren Stufen umfaßt, in denen man die Lösung mit TNBP, jedoch ohne UV-Bestrahlung oder β-Propiolacton, bei einem pH-Wert von 3,0 bis 5,0 unter Bedingungen in Kontakt bringt, die hinreichen, eine wesentliche Herabsetzung der Viren ohne gegenläufige Auswirkung auf die Proteine zu gewährleisten.

2. Verfahren gemäß Anspruch 1, wobei die Lösung mit TNBP und einem Detergens in Kontakt gebracht wird.

3. Verfahren gemäß Anspruch 2, worin das Detergens Polysorbat 80 ist.

4. Verfahren gemäß Anspruch 1, wobei der pH-Wert ca. 4,0 bis ca. 4,85 beträgt.

5. Verfahren gemäß Anspruch 1, worin die Proteine Antikörper sind.

6. Verfahren gemäß Anspruch 5, worin die Antikörper vom IgM- oder vom IgG-Typ sind.

7. Verfahren gemäß Anspruch 1, wobei die Herabsetzung der Viren mehr als 2 log-Einheiten ausmacht.

8. Verfahren gemäß Anspruch 1, wobei die gegenläufigen Auswirkungen auf die biologische Wirksamkeit der Proteine weniger als 30% ausmachen.

## Revendications

1. Procédé pour inactiver des virus dans une solution aqueuse de protéines thérapeutiques biologiquement actives, comprenant les étapes consistant à mettre en contact la solution avec du TNBP mais sans irradiation UV ou β-propiolactone, à un pH compris dans la plage de 3,0 à 5,0 dans des conditions suffisantes pour garantir une réduction importante des virus sans effet néfaste sur les protéines.

2. Procédé suivant la revendication 1, dans lequel la solution est mise en contact avec du TNBP et un détergent.

3. Procédé suivant la revendication 2, dans lequel le détergent consiste en polysorbate 80.

4. Procédé suivant la revendication 1, dans lequel le pH est compris dans la plage d'environ 4,0 à 4,85.

5. Procédé suivant la revendication 1, dans lequel les protéines sont des anticorps.

6. Procédé suivant la revendication 5, dans lequel les anticorps sont du type IgM ou IgG.

7. Procédé suivant la revendication 1, dans lequel la réduction des virus est supérieure à 2 logs.

8. Procédé suivant la revendication 1, dans lequel les effets néfastes sur l'activité biologique des protéines sont inférieurs à 30 %.
